(19) Europäisches Patentamt
European Patent Office
Office européen des brevets

(11) Numéro de publication : **0 325 537 B1**

# (12) FASCICULE DE BREVET EUROPEEN

(45) Date de publication du fascicule du brevet : **07.04.93 Bulletin 93/14**

(51) Int. Cl.$^5$ : **C07H 19/073**

(21) Numéro de dépôt : **89400155.1**

(22) Date de dépôt : **19.01.89**

## (54) Procédé de synthèse de l'azido-3'-desoxy-3'-thymidine et analogues.

(30) Priorité : **19.01.88 FR 8800553**
**09.12.88 FR 8816248**

(43) Date de publication de la demande : **26.07.89 Bulletin 89/30**

(45) Mention de la délivrance du brevet : **07.04.93 Bulletin 93/14**

(84) Etats contractants désignés :
**AT BE CH DE ES FR GB GR IT LI LU NL SE**

(56) Documents cités :
**EP-A- 0 199 451**
**US-A- 4 681 933**
**CHEMICAL ABSTRACTS, vol. 92, 1980, page 716, résumé no. 198690r, Columbus, Ohio, US; K.A. WATANABE et al.: "2,5'-anhydrouridine and 2,5'-anhydro-5-fluorouridine. One-step conversion of uridine and 5-fluorouridine into their corresponding 2,5'-anhydronucleosides", NUCL. ACID CHEM. 1978, 1, 343-6**
**JOURNAL OF ORGANIC CHEMISTRY, vol. 35, no. 9, 1970, pages 2868-2877; J.P.H. VERHEYDEN et al.: "Halo sugar nucleosides. II. Iodination of secondary hydroxyl groups of nucleosides with methyltriphenoxyphosphonium iodide"**
**CHEMICAL ABSTRACTS, vol. 91, 1979, page 623, résumé no. 74826z, Columbus, Ohio, US; J. KIMURA et al.: "Studies on nucleosides and nucleotides. VII. Preparation of pyrimidine nucleoside 5'-phosphates and N3,5'-purine cyclonucleosides by selective activation of the 5'-hydroxyl group", & BULL. CHEM. SOC. JPN. 1979, 52(4), 1191-6**
**CHEMICAL ABSTRACTS, vol. 101, no. 21, novembre 1984, page 813, résumé no. 192378c, Columbus, Ohio, US; V.E. ZAITSEVA et al.: "Aminonucleosides and their derivatives. XI. Synthesis of 3'-amino-2',3'-dideoxynucleoside 5'-triphosphates", & BIOORG. KHIM, 1984, 10(5), 670-80**

(56) Documents cités :
**JOURNAL OF THE CHEMICAL SOCIETY, PERKIN TRANSACTIONS I, pages 306-310, Londres, GB; I. YAMAMOTO et al.: "One-step synthesis of 5'-azido-nucleosides"**

(73) Titulaire : **UNIVERSITE PIERRE ET MARIE CURIE PARIS VI**
**4, place Jussieu**
**F-75230 Paris Cedex 05 (FR)**

(72) Inventeur : **Czernecki, Stanislas**
**5 c. des Bertagnes Rubelles**
**F-77950 Maincy (FR)**
Inventeur : **Valery, Jean-Marc**
**250, Rue de la Faisanderie**
**F-77176 Nandy (FR)**
Inventeur : **Ville, Guy**
**15 c. Ch. Friedel**
**F-75020 Paris (FR)**

(74) Mandataire : **Ahner, Francis et al**
**CABINET REGIMBEAU, 26, avenue Kléber**
**F-75116 Paris (FR)**

EP 0 325 537 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de synthèse de l'azido-3 -désoxy-3′-thymidine (azido-thymide, AZT, zidovudine) et de composés apparentés utilisés dans la lutte contre le SIDA.

Une autorisation de mise sur le marché à été récemment accordée à la firme Wellcome pour la commercialisation d'un produit à base d'azido-thymidine. L'application thérapeutique de ce produit est décrite dans le brevet DE-36 08 606.

Différentes synthèses de l'AZT ont été décrites à ce jour, elles ont en commun de partir de la thymidine et de comporter un assez grand nombre d'étapes, entre cinq et sept, sauf une qui nécessite l'utilisation d'une base coûteuse.

Ces synthèses sont résumées, par exemple, dans l'article de Drugs of the Future, vol. 11, n° 12 (1986), les rendements de la transformation sont très faibles dans tous les cas.

Un article publié en 1984 (V.E. Zaitseva et coll., Bioorg. Khim., 10, 670 (1984)) décrivait une synthèse de l'AZT mettant en oeuvre trois étapes, il semble, néanmoins, que ce procédé n'ait pas été développé.

En effet, la seconde étape du procédé nécessite la préparation de p-méthyl-benzoate de lithium et l'isolement de l'AZT obtenu dans la dernière étape se fait par chromatographie sur colonne.

La présente invention a pour objet un procédé de préparation d'AZT et de composés apparentés qui ne met en oeuvre que deux étapes et qui, en outre, présente l'énorme avantage de pouvoir être conduit dans un seul récipient réactionnel, si besoin est.

Plus particulièrement, il s'agit d'un procédé de préparation d'un composé de formule (I) :

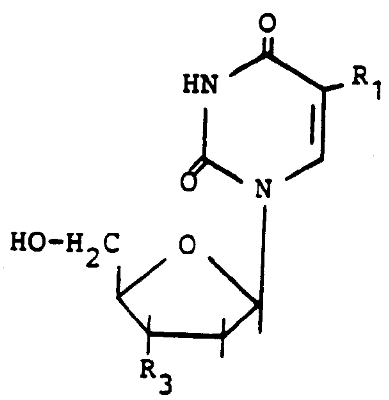

I

dans laquelle $R_1$ est H, un radical alkyle, un radical alcoxy, hydroxyalkyle ou halogène, et $R_3$ est $N_3$ ou un radical CN,
caractérisé en ce qu'on fait réagir un composé de formule (II) :

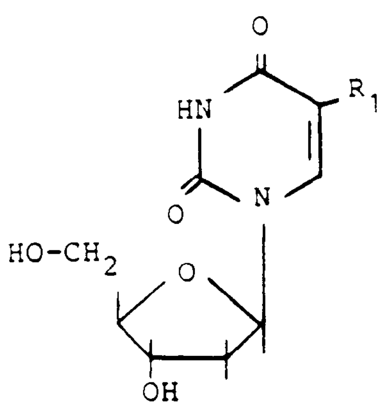

II

avec un dérivé de phosphine ou de phosphite et un diester de l'acide azo-dicarboxylique et d'un acide carboxylique $R_2$-COOH dans un solvant compatible avec les conditions de réaction pour former le composé de formule (III) :

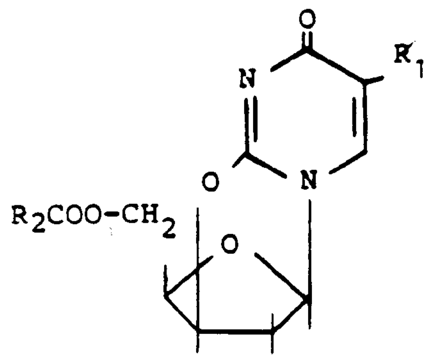

III

lequel, après une éventuelle séparation, est ouvert en présence d'un azoture ou d'un cyanure dans un solvant compatible avec les conditions de réaction, le composé de formule (I) est ensuite isolé du milieu réactionnel, après déprotection de la position 5'.

L'article du CHEMICAL ABSTRACTS, vol. 92, 1980, page 716, résumé n° 198690r, Columbus, Ohio, US; K.A. WATANABE et al., décrit la conversion de l'uridine en $0^2$, 5' anhydro-uridine à partir de $PPH_3$ et DEAD dans du dioxane.

Le document EP 199 451 décrit l'ouverture d'un l'$O^{2,}$ 3'-cyclonucléoside en présence d'azoture alcalin.

Le document JOURNAL OF ORGANIC CHEMISTRY, vol. 35, n° 9, 1970, pages 2868-77; J.P.H. VERHEYDEN et al. décrit la protection de l'hydroxyl en 5' de la thymidine sous forme d'ester et l'activation de l'hydroxyl en 3' en vue d'être converti en $O^2$, 3'-anhydro-thymidine.

La préparation d'un composé de formule III avec $R_1$ représentant un groupement méthyle et $R_2$ un groupement phényle a été citée dans la littérature (KIMURA et al. Bull. of the Chem. Soc. of Japan Vol. 52 (4), 1979, p. 1191) mais le procédé en cause n'est pas satisfaisant car il nécessite deux étapes et conduit à un faible rendement, de l'ordre de 35 %.

Bien entendu, parmi les composés de formule (I), il faut citer plus particulièrement l'AZT, c'est-à-dire le composé dans lequel $R_1$ est égal à $CH_3$ et $R_3$ est égal à $N_3$ ; néanmoins, actuellement, d'autres dérivés de l'AZT sont développés, il s'agit en particulier des dérivés dans lesquels $R_1$ est égal à un radical alkyle inférieur, un radical alcoxy inférieur ou hydroxyalcoyle inférieur, comportant de 1 à 5 atomes de carbone, par exemple des radicaux méthyle ou éthyle.

Dans le procédé selon la présente invention, le produit de départ, en particulier pour préparer l'AZT, est la thymidine, composé qui est disponible industriellement et qui doit, de préférence, pour les besoins du procédé être en général déshydraté car la présence d'eau nuit considérablement au rendement du procédé.

Parmi les autres réactifs utilisés pour la préparation d'un composé de formule III, bien qu'il soit possible d'utiliser différents types de phosphine ou de phosphite, la phosphine préférée est la triphénylphosphine car il s'agit d'un produit qui est facilement accessible industriellement. De même les diesters d'acide azo-dicarboxylique peuvent être des esters d'alkyle ou d'aryle, en particulier des esters d'alkyle inférieur, tel l'azodicarboxylate de diéthyle (DEAD) ou de préférence l'azodicarboxylate de diisopropyle (DIAD). Ce composé DIAD est en effet un composé qui est peu coûteux et qui permet d'obtenir des rendements égaux ou même parfois supérieurs à ceux obtenus avec les autres diesters.

Enfin, dans le choix de l'acide carboxylique, $R_2$-COOH, $R_2$ représente un radical aliphatique et ses dérivés ou de préférence un radical aromatique et ses dérivés. Par radical aromatique, on entend des radicaux tels phényle, indényle ou encore des hétérocycles aromatiques à 5 atomes tel furyle.

Ces radicaux pourront être également substitués ( une ou plusieurs fois) par des groupements hétéroatomiques tels halogènes, alcoyloxy ou nitro.

Ainsi, l'utilisation à titre d'acide carboxylique $R_2$-COOH, de l'acide benzoïque, selon la présente invention, permet une excellente protection de la fonction OH dans d'excellentes conditions de rendement.

En outre, la présence de ce groupement en 5' favorise l'ouverture du cycle par un azoture alcalin lors de la seconde étape du procédé. Il permet également dans le cas où l'on souhaite isoler le composé de formule (III), d'obtenir un produit insoluble ou du moins un produit qui peut être facilement précipité à partir du solvant de réaction, en utilisant par exemple un tiers solvant tel que de l'éther ou un ester, ou dans des conditions réactionnelles favorisant la précipitation telles que faible volume de solvant et/ou utilisation d'une basse température lors de la séparation.

Les conditions de température et de pression de la réaction ne sont pas, à proprement parler, caractéristiques. En effet, la réaction peut être effectuée à la température ordinaire et à pression ordinaire.

La durée de réaction dépend, bien entendu, des paramètres précis mis en oeuvre, mais la plupart du temps

elle est complète au bout de quelques heures, bien qu'on puisse la laisser davantage sans aucun inconvénient.

A la fin de cette première étape réactionnelle, on obtient le composé de formule (III) qui peut être, soit séparé du mélange réactionnel comme cela a été dit précédemment, avant de subir la seconde étape, ou bien, au contraire, le mélange réactionnel peut être directement traité, comme cela sera décrit ci-après.

Le solvant de la réaction doit être un solvant compatible avec les conditions de réaction. Il s'agira la plupart du temps d'un solvant aprotique polaire anhydre, comme cela a été indiqué précédemment, la présence d'eau peut, en effet, nuire gravement au rendement du procédé.

Parmi ces solvants, il faut citer le DMF, l'HMPT, le DMSO, pour des raisons de coût, le DMF est le solvant préféré compte tenu du fait qu'il permet, en outre, la mise en oeuvre de la seconde étape du procédé sans avoir à séparer le produit formé dans la première.

Les différents réactifs sont utilisés de préférence en excès molaire par rapport au composé de formule (II).

Lors de la seconde étape de préparation, la réaction d'ouverture du cycle est effectuée en présence d'un excès d'azoture ou de cyanure. Il est possible dans certains cas de diminuer l'excès de réactif en faisant la réaction en présence d'un acide de Lewis ou un autre sel de lithium. Cette réaction sera de préférence réalisée en présence seulement d'un léger excès d'azoture par mole de composé de formule III, c'est-à-dire 1,5 équivalent.

Le solvant utilisé doit permettre la solubilisation à la fois du composé III et de l'azoture ou du cyanure utilisé. Ce sera en général un solvant aprotique polaire anhydre comme cité précédemment.

Ainsi, la réaction pourra être effectuée dans le DMF à chaud afin de favoriser la solubilisation, par exemple à des températures de l'ordre de 100°C jusqu'à la température de reflux qui devront rester compatibles avec la stabilité des produits mis en jeu, la réaction sera alors complète en quelques heures, c'est-à-dire environ entre 7 et 10 heures.

Les azotures alcalins ou les cyanures alcalins peuvent être utilisés dans cette étape réactionnelle.

La séparation du composé de formule (I) obtenu peut être effectuée selon deux méthodes.

La première consiste à l'isoler du mélange réactionnel après avoir traité celui-ci avec un hydrogéno-carbonate alcalin, par exemple par extraction avec un solvant halogéné tel que le chloroforme.

La phase organique est alors lavée à l'eau, séchée et évaporée afin d'obtenir un produit homogène. Ce composé est alors saponifié puis extrait.

Il est, bien entendu, possible directement à partir du milieu réactionnel de l'étape 2 de saponifier le produit, puis, après séparation des différents produits organiques et des différents sels présents, de récupérer le composé de formule (I), en particulier l'AZT, de façon quasi quantitative.

Les exemples ci-après permettront de mettre en évidence d'autres avantages et caractéristiques de la présente invention.

**Exemple 1**

A 10 mMoles de thymidine et 15 mMoles de $(Ph)_3P$, dans 25 ml de DMF anhydre, on ajoute goutte à goutte, à la température ordinaire (refroidir pour une plus grande quantité) le mélange suivant :

15 mMoles de PhCOOH + 15 mMoles de DEAD dans 25 ml de DMF anhydre. La chromatographie sur couche mince (c.c.m.) (éluant A : AcOEt 20, MeOH 1) indique que la réaction est terminée en 30 minutes environ. La position 5′ est alors benzoylée.

On ajoute ensuite successivement 15 mMoles de $(Ph)_3P$ et 15 mMoles de DEAD (lentement). On laisse agiter à la température ordinaire. La réaction évolue peu après 3 heures, mais on peut laisser davantage sans inconvénient. La c.c.m. (éluant B : $CHCl_3$ 4, EtOH 1) montre la présence de (III) plus polaire que la thymidine et des sous-produits de la réaction (très peu polaires).

L'isolement du composé (III) se fait très facilement en versant le mélange réactionnel (la présence d'un éventuel précipité ne gène pas) dans 250 ml d'éther sous agitation. Le précipité blanc est essoré et lavé à l'éther. On obtient 2,803 de (III) (85,5 %).

(Tf. 242°C; 1 tache en c.c.m. (éluant B) ; I.R. et $^1H$ RMN en accord avec la structure).

Le traitement des eaux-mères (extraction ou cristallisation) permet d'obtenir davantage de composé (III) (10-13 %). Le produit ainsi préparé est d'une pureté satisfaisante pour effectuer la seconde étape.

Exemple 2

2,46 g de (III) sont traités par 1,5 g de $LiN_3$ (4 équivalents) dans 20 ml de DMF à reflux. Lorsque la réaction est terminée, le mélange résultant est versé dans une solution saturée d'hydrogéno-carbonate de sodium (70 ml) et le solide blanc est extrait au chloroforme (3 x 20 ml). Après lavage à l'eau, séchage et évaporation du

solvant, on obtient 2.7 g (96 %) de produit homogène en c.c.m.

Ce composé est directement saponifié à l'aide de 1,15 équivalent de MeONa dans 50 ml de MeOH. Lorsque la réaction est terminée, le mélange est versé dans 30 ml d'eau et le méthanol est évaporé. La solution aqueuse est extraite à l'éther (2 x 20 ml) pour éliminer le benzoate de méthyle. On ajoute environ 5 g de résine $H^+$ (Amberlite IRN-77) et agite à l'ambiante. Lorsque le pH est neutre, la solution est filtrée et le solvant évaporé. On obtient l'AZT de façon quantitative (1,940 g, environ 100%). Le composé obtenu est homogène en c.c.m. et son spectre de $^1H$ RMN est en accord avec sa structure.

EXEMPLE 3

A 2 mMoles de thymidine et 3 mMoles de $(Ph)_3P$ dans 5 ml de DMF anhydre, on ajoute goutte à goutte, à la température ordinaire le mélange suivant :

3 mMoles d'un acide carboxylique aromatique $R_2COOH$ + 3 mMoles de DEAD dans 5 ml de DMF anhydre.

Dans le cas où l'acide $R_2COOH$ n'est pas soluble dans le DMF, il est introduit dans le milieu en même temps que la thymidine.

La c.c.m. (éluant A : AcOEt 20, MeOH 1) indique que la réaction est terminée en 30 minutes.

On ajoute alors successivement 3 mMoles de $(Ph)_3P$ et 3 mMoles de DEAD (goutte à goutte). On laisse agiter 2 heures à température ordinaire. Dans le cas où un précipité est observé, le solide est essoré, lavé à l'éther et séché. Sinon on procède comme dans l'exemple 1.

Les résultats sont présentés dans le tableau ci-dessous

| $R_2$ | Quantité (rendement) | F (°C) |
|---|---|---|
| 2 - furyle | 483 mg (76 %) | 260 |
| 2 - bromophényle | 526 mg (64,5 %) | 238 |
| 4 - bromophényle | 486 mg* (59,5 %) | 300 |
| 4 - nitrophényle | 494 mg* (66 %) | 300 |
| 3,5 - dinitrophényle | 758 mg (90,5 %) | 258 |
| 4 - méthoxyphényle | 546 mg* (82 %) | 260 |
| 3,4 - diméthoxyphényle | 660 mg (84,5 %) | 240 |
| 2,6 - dichlorophényle | 602 mg (76 %) | 222 |

* première fraction obtenue en procédant comme dans l'exemple 4.

EXEMPLE 4 :

A 5 mMoles de thymidine et 7,5 mMoles de $(Ph)_3P$ dans 12,5 ml de DMF, on ajoute goutte à goutte, à la température ordinaire, le mélange suivant :

7,5 mMoles de PhCOOH et 7,5 mMoles de DIAD (azodicarboxylate de diisopropyle) dans 12,5 ml de DMF. La c.c.m. (éluant A : AcOEt 20, MeOH 1) indique que la benzoylation de la position 5' est terminée en 30 minutes.

On ajoute ensuite successivement 7,5 mMoles de $(Ph)_3P$ et 7,5 mMoles de DIAD (goutte à goutte). On laisse agiter à la température ordinaire. La réaction évolue peu après 2 heures, mais on peut laisser davantage sans inconvénient. La c.c.m. (éluant B : $CHCl_3$4, EtOH1) montre la présence de (III) et des sous-produits de la réaction (très peu polaires).

L'isolement du composé (III) se fait, en versant le mélange réactionnel dans 125 ml d'éther sous agitation. Le précipité blanc est essoré et lavé par 25 ml d'éther.

Le composé (III) est obtenu avec un rendement de 83 %.

De la même façon, l'isolement du composé (III) peut se faire en versant le mélange réactionnel dans 125 ml d'acétate de butyle. Le composé (III) sera alors obtenu avec un rendement de 73 %.

EXEMPLE 5 :

A une solution de 5 mMoles de thymidine, de 7,5 mMoles de $(Ph)_3P$ et de 7,5 mMoles de PhCOOH dans 12,5 ml de DMF, on ajoute goutte à goutte 7,5 mMoles de DIAD.

Après 30 minutes environ la c.c.m. (éluant A : AcOEt 20, MeOH 1) indique que la thymidine a disparu.

On ajoute alors successivement 7,5 mMoles de $(Ph)_3P$ et 7,5 mMoles de DIAD (goutte à goutte). On laisse agiter 1 heure à la température ordinaire et on filtre le précipité formé. Le solide est lavé par 25 ml d'éther et séché.

On obtient 0,955 g de (III) (58,2 %). Une deuxième fraction de (III) est obtenue en versant les eaux-mères dans 125 ml d'éther sous agitation. La filtration fournit 0,24 g de (III) (14,6 %).

La quantité totale de (III) obtenue est de 1,195 g (73 %).

EXEMPLE 6 :

2,434 g de (III:$R_2$=Ph) sont dissous dans 15 ml de DMF de 140°C. On ajoute alors 544 mg de $LiN_3$ (1,5 équivalents). Après 4h de chauffage à cette température, la réaction est terminée. Le mélange résultant est dilué avec 40 ml de chloroforme, la solution est extraite avec 75 ml de solution aqueuse saturée en hydrogénocarbonate de sodium. La solution aqueuse est extraite au chloroforme (3 x 35 ml). Après lavage à l'eau, séchage et évaporation du solvant, on obtient l'AZT benzoylée en 5′ de façon quantitative. Le composé est homogène en c.c.m. et contient des traces de DMF qui ne gènent pas dans l'étape suivante.

EXEMPLE 7 :

2g de (III:$R_2$ =Ph) sont traités par 2,7 g de $LiN_3$ (9 équivalents) dans 16 ml de DMF anhydre à 110°C. Lorsque la réaction est terminée, le mélange résultant est traité comme dans l'exemple 3. On obtient ainsi 2,509g de produit homogène en c.c.m. dont le spectre de 1H RMN (90 $MH_Z$) indique la présence de 12 % de DMF. La transformation est donc quantitative.

EXEMPLE 8 :

2,1 g de benzoyl-5′- azido-3′-thymidine (contenant 8,5 % de DMF) sont dissous à chaud dans 20 ml de méthanol. On ajoute ensuite 1,15 équivalent de MeONa molaire dans le méthanol (6,6 ml). La solution est abandonnée à la température ordinaire. Lorsque la réaction est terminée, le mélange est versé dans 30 ml d'eau et le méthanol est évaporé. La solution aqueuse est extraite à l'éther (2 x 20 ml) pour éliminer le benzoate de méthyle. La solution est ensuite neutralisée à l'aide d'environ 2 g de résine Amberlite-IRN-77 ($H^+$). Après filtration et évaporation du solvant, on obtient 1,424 g (94 %) d'AZT.

EXEMPLE 9 :

2,1 g de benzoyl-5′- azido-3′-thymidine (contenant 8,5 % de DMF) sont traités par 6,6 ml de MeONa molaire dans le méthanol comme dans l'exemple 8. Lorsque la réaction est terminée, on évapore le méthanol et ajoute 50 ml d'eau. Le mélange est soumis à un entrainement à la vapeur d'eau pour éliminer le benzoate de méthyle. La phase aqueuse est ensuite neutralisée et traitée comme dans l'exemple 5. On obtient 1,422 g (94 %) d'AZT homogène en c.c.m.

EXEMPLE 10 :

2,434 g de (III:$R_2$=Ph) sont traités par 1,5 équivalents de $LiN_3$, comme dans l'exemple 6. Le produit résultant qui contient des traces de DMF est traité par 20 ml d'eau et 7,5 ml de soude 3 M dans un mélange méthanol-eau (1 : 1). Le mélange est agité à température ordinaire. Lorsque la réaction est terminée la solution est neutralisée avec la quantité calculée d'HCl concentré. La solution est saturée à l'aide de NaCl et extraite à l'acétate d'éthyle (3 x 30 ml). La phase organique est séchée et le solvant évaporé après filtration. On obtient 2,164 g de produit brut qui contient des traces de DMF et d'AcoEt, un peu d'acide benzoïque ($^1$H RMN) et au moins 1,78 g d'AZT.

EXEMPLE 11 :

1,19 g de (III : $R_2$=Ph) sont dissous dans 5 ml de DMF à 140°C. On ajoute alors 470 mg de $NaN_3$ (2 équivalents). Après 7 heures de chauffage à cette température, la réaction est terminée. Le mélange est traité comme dans l'exemple 6. On obtient 1,439 g d'un produit homogène en c.c.m. contenant 7 % de DMF. Le transformation est quantitative.

## Revendications

1. Procédé de préparation d'un composé de formule (I) :

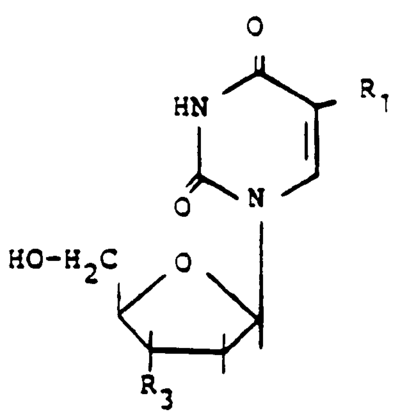

I

dans laquelle $R_1$ est H, un radical alkyle, un radical alcoxy, hydroxyalkyle ou halogène, et $R_3$ est $N_3$, un radical CN,
caractérisé en ce qu'on fait réagir un composé de formule (II) :

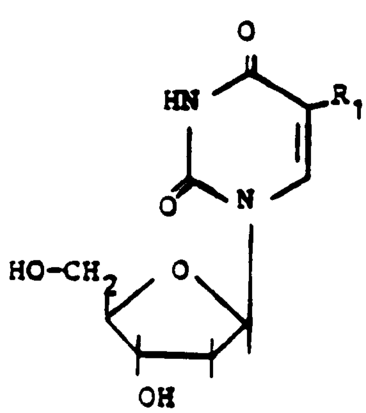

II

avec un dérivé de phosphine et un diester d'acide azo-dicarboxylique et d'un acide carboxylique $R_2$-COOH dans un solvant compatible avec les conditions de réaction pour former le composé de formule (III) :

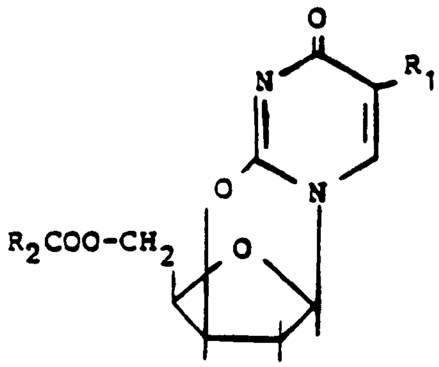

III

lequel, après une éventuelle séparation, est ouvert en présence d'un azoture ou d'un cyanure dans un solvant compatible avec les conditions de réaction, le composé de formule (I) est ensuite isolé du milieu réactionnel, après déprotection de la position 5′, l'acide carboxylique $R_2COOH$ étant un acide aliphatique ou aromatique substitué ou non substitué.

2. Procédé selon la revendication 1, caractérisé en ce que l'acide carboxylique $R_2COOH$ est l'acide benzoïque ou un dérivé substitué de l'acide benzoïque.

3. Procédé selon l'une des revendications 1 à 2, caractérisé en ce que l'acide carboxylique $R_2$ COOH est un dérivé de l'acide benzoïque substitué par un ou plusieurs groupements halogène, alcoxyloxy ou nitro.

4. Procédé selon l'une des revendications 1 à 3, caractérisé en ce que la phosphine utilisée est la triphénylphosphine.

5. Procédé selon l'une des revendications 1 à 4, caractérisé en ce que le diester de l'acide azo-dicarboxylique est un ester d'alkyle ou d'aryle.

6. Procédé selon la revendication 5, caractérisé en ce qu'il s'agit d'un ester d'alkyle inférieur.

7. Procédé selon l'une des revendications 1 à 6, caractérisé en ce que le solvant de réaction est un solvant aprotique polaire anhydre.

8. Procédé selon la revendication 7, caractérisé en ce que le solvant est choisi parmi le DMF, l'HMPT, le DMSO.

9. Procédé selon l'une des revendications 1 à 8, caractérisé en ce que le produit (III) est séparé du mélange réactionnel par précipitation.

10. Procédé selon la revendication 9, caractérisé en ce que la précipitation du composé (III) peut être obtenue en ajoutant un éther ou un ester au milieu réactionnel.

11. Procédé selon l'une des revendications 1 à 9, caractérisé en ce que les réactifs sont utilisés en excès molaire par rapport au composé (II).

12. Procédé selon l'une des revendications 1 à 8, caractérisé en ce que le composé de formule (III) n'est pas séparé du milieu réactionnel.

13. Procédé selon l'une des revendications 1 à 12, caractérisé en ce que le composé de formule (III) est traité par un excès d'azoture ou de cyanure dans un solvant aprotique polaire.

14. Procédé selon l'une des revendications 1 à 12, caractérisé en ce que le composé de formule (III) est traité par 1,5 équivalent d'azoture alcalin dans le DMF.

15. Procédé selon l'une des revendications 1 à 14, caractérisé en ce que le produit est récupéré après saponification et neutralisation.

**Patentansprüche**

1. Verfahren zur Herstellung einer Verbindung der Formel (I):

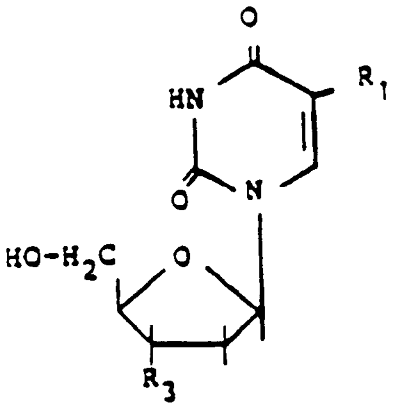

I

in der $R_1$ Wasserstoff, einen Alkylrest, einen Alkoxyrest, einen Hydroxyalkylrest oder ein Halogen darstellt und $R_3$ $N_3$ oder einen CN-Rest bedeutet, dadurch gekennzeichnet, daß man eine Verbindung der Formel (II):

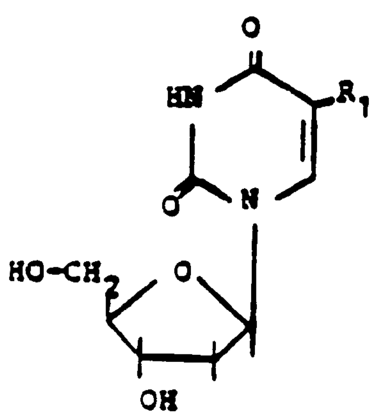

II

mit einem Phosphin-Derivat und einem Diester der Azodicarbonsäure und einer Carbonsäure $R_2$-COOH in einem geeigneten Lösungsmittel und unter Reaktionsbedingungen zur Reaktion bringt, um die Verbindung der Formel (III):

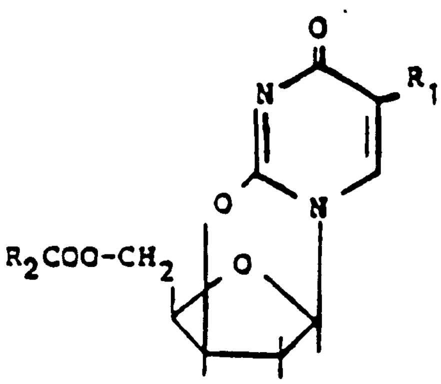

III

zu bilden, die nach einer eventuellen Abtrennung in Anwesenheit eines Azids oder Cyanids in einem geeigneten Lösungsmittel unter den Reaktionsbedingungen geöffnet wird, wonach man die Verbindung der Formel (I) aus dem Reaktionsmedium isoliert und die Abspaltung der Schutzgruppe in Position 5′ vornimmt, wobei die Carbonsäure $R_2COOH$ eine substituierte oder nicht substituierte, aliphatische oder aromatische Säure ist.

2. Verfahren nach Anspruch 1, dadurch gekennzeichnet, daß die Carbonsäure $R_2COOH$ Benzoesäure oder ein substituiertes Derivat der Benzoesäure ist.

3. Verfahren nach einem der Ansprüche 1 bis 2, dadurch gekennzeichnet, daß die Carbonsäure $R_2COOH$ ein durch ein oder mehrere Halogen-, Alkyloxy- oder Nitrogruppen substituiertes Benzoesäure-Derivat ist.

4. Verfahren nach einem der Ansprüche 1 bis 3, dadurch gekennzeichnet, daß das verwendete Phosphin Triphenylphosphin ist.

5. Verfahren nach einem der Ansprüche 1 bis 4, dadurch gekennzeichnet, daß der Diester der Azodicarbonsäure ein Alkyl- oder Arylester ist.

6. Verfahren nach Anspruch 5, dadurch gekennzeichnet, daß es sich um einen niederen Alkylester handelt.

7. Verfahren nach einem der Ansprüche 1 bis 6, dadurch gekennzeichnet, daß das Lösungsmittel der Reaktion ein wasserfreies, polares aprotisches Lösungsmittel ist.

8. Verfahren nach Anspruch 7, dadurch gekennzeichnet, daß das Lösungsmittel unter DMF, HMPT und DMSO ausgewählt wird.

9. Verfahren nach einem der Ansprüche 1 bis 8, dadurch gekennzeichnet, daß das Produkt (III) von der Reaktionsmischung durch Fällung getrennt wird.

10. Verfahren nach Anspruch 9, dadurch gekennzeichnet, daß die Fällung der Verbindung (III) durch Zugabe eines Ethers oder eines Esters zum Reaktionsmedium erhalten werden kann.

11. Verfahren nach einem der Ansprüche 1 bis 9, dadurch gekennzeichnet, daß die Reaktanten im molaren Überschuß in bezug auf die Verbindung (II) verwendet werden.

12. Verfahren nach einem der Ansprüche 1 bis 8, dadurch gekennzeichnet, daß die Verbindung der Formel (III) nicht vom Reaktionsmedium abgetrennt wird.

13. Verfahren nach einem der Ansprüche 1 bis 12, dadurch gekennzeichnet, daß die Verbindung der Formel (III) mit einem Überschuß von Azid oder Cyanid in einem polaren aprotischen Lösungsmittel behandelt wird.

14. Verfahren nach einem der Ansprüche 1 bis 12, dadurch gekennzeichnet, daß die Verbindung der Formel (III) mit 1,5 Äquivalenten Alkali-Azid in DMF behandelt wird.

15. Verfahren nach einem der Ansprüche 1 bis 14, dadurch gekennzeichnet, daß das Produkt nach Verseifung und Neutralisation gewonnen wird.

## Claims

1. Process for preparing a compound of formula (I)

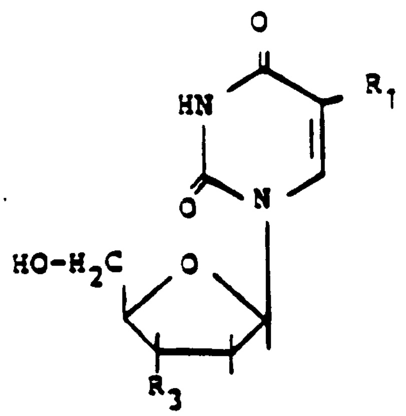

I

in which $R_1$ is H, an alkyl radical or an alkoxy, hydroxyalkyl or halogen radical, and $R_3$ is $N_3$ or a CN radical, characterized in that a compound of formula (II):

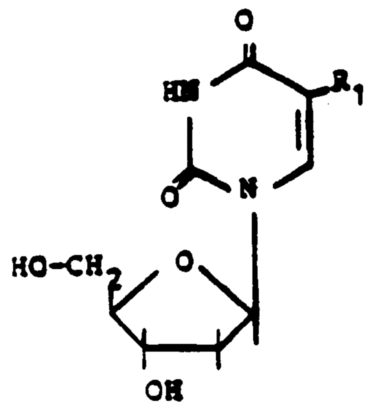

II

in reacted with a phosphine derivative and an azodicarboxylic acid diester and a carboxylic acid $R_2$-COOH in a solvent compatible with the reaction conditions, to form the compound of formula (III):

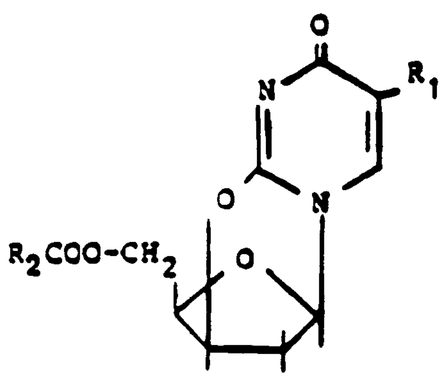

III

which, after separation if required, is opened in the presence of an azide or a cyanide in a solvent compatible with the reaction conditions, and the compound of formula (I) is then isolated from the reaction medium after deprotection of the 5′-position, the carboxylic acid $R_2COOH$ being a substituted or unsubstituted aliphatic or aromatic acid.

2. Process according to Claim 1, characterized in that the carboxylic acid $R_2COOH$ is benzoic acid or a substituted derivative of benzoic acid.

3. Process according to one of Claims 1 to 2, characterized in that the carboxylic and $R_2COOH$ is a derivative

of benzoic acid substituted with one or more halogen, alkyloxy or nitro groups.

4. Process according to one of Claims 1 to 3, characterized in that the phosphine used is triphenylphosphine.

5. Process according to one of Claims 1 to 4, characterized in that the azodicarboxylic acid diester is an alkyl or aryl ester.

6. Process according to Claim 5, characterized in that the diester is a lower alkyl ester.

7. Process according to one of Claims 1 to 6, characterized in that the reaction solvent is an anhydrous polar aprotic solvent.

8. Process according to Claim 7, characterized in that the solvent is chosen from DMF, HMPT and DMSO.

9. Process according to one of Claims 1 to 8, characterized in that the product (III) is separated from the reaction mixture by precipitation.

10. Process according to Claim 9, characterized in that the precipitation of the compound (III) can be obtained by adding an ether or an ester to the reaction medium.

11. Process according to one of Claims 1 to 9, characterized in that the reactants are used in molar excess relative to the compound (II).

12. Process according to one of Claims 1 to 8, characterized in that the compound of formula (III) is not separated from the reaction medium.

13. Process according to one of Claims 1 to 12, characterized in that the compound of formula (III) is treated with an excess of azide or of cyanide in a polar aprotic solvent.

14. Process according to one of Claims 1 to 12, characterized in that the compound of formula (III) is treated with 1.5 equivalents of alkali metal azide in DMF.

15. Process according to one of Claims 1 to 14, characterized in that-the product is recovered after saponification and neutralization.